(19) Europäisches Patentamt
European Patent Office
Office européen des brevets

(11) **EP 1 743 634 A1**

(12) **EUROPEAN PATENT APPLICATION**
published in accordance with Art. 158(3) EPC

(43) Date of publication:
**17.01.2007 Bulletin 2007/03**

(21) Application number: **05737248.4**

(22) Date of filing: **02.05.2005**

(51) Int Cl.:
*A61K 31/198* (2006.01)     *A23L 1/305* (2006.01)
*A23L 2/02* (2006.01)     *A61P 3/00* (2006.01)

(86) International application number:
**PCT/JP2005/008283**

(87) International publication number:
**WO 2005/107734 (17.11.2005 Gazette 2005/46)**

(84) Designated Contracting States:
**AT BE BG CH CY CZ DE DK EE ES FI FR GB GR HU IE IS IT LI LT LU MC NL PL PT RO SE SI SK TR**

(30) Priority: **06.05.2004 JP 2004136960**

(71) Applicant: **Taiyokagaku Co., Ltd.**
**Yokkaichi-shi, Mie 510-0825 (JP)**

(72) Inventors:
• **OKUBO, Tsutomu**
  **Yokkaichi-shi**
  **Mie 5100825 (JP)**
• **OZEKI, Makoto**
  **Yokkaichi-shi**
  **Mie 5100825 (JP)**

• **SADZUKA, Yasuyuki**
  **School  Pharmaceutical Sciences**
  **Shizuoka-shi**
  **Shizuoka 4228002 (JP)**
• **JUNEJA, Lekh Raj**
  **Yokkaichi-shi**
  **Mie 5100825 (JP)**

(74) Representative: **Paget, Hugh Charles Edward et al**
  **Mewburn Ellis LLP**
  **York House**
  **23 Kingsway**
  **London WC2B 6HP (GB)**

(54) **ALCOHOL METABOLISM ACCELERATING COMPOSITION, AND FOOD OR DRINK CONTAINING THE COMPOSITION**

(57)    An alcohol-metabolism enhancing composition contains theanine. The composition may be contained in ingesta. Theanine has an effect of quickly reducing blood alcohol concentration, thereby easing or improving trou- bles due to alcoholic ingestion such as hangover or al- coholic hepatic insufficiency.

EP 1 743 634 A1

**Description**

BACKGROUND OF THE INVENTION

1. Field of the invention

The present invention relates to an alcohol-metabolism enhancing composition containing theanine and ingesta containing the composition.

2. Description of the related art

Consumption of alcohol has been increasing year by year in Japan. The National Tax Agency's annual report describes that net consumption of alcohol amounted to 869, 889 kiloliter in 1997. The number of adult drinkers is said to amount to about 66 million. Accordingly, net consumption of alcohol per adult drinker is about 8.8 liter a year. It is known that ingestion of excessive alcohol causes alcoholic insufficiency accompanying reduction in glutathione (GSH) or increase in lipid peroxide in the liver. Therefore, it is desirable to reduce an amount of alcoholic ingestion in order that alcoholic sickness may be reduced.

However, a proper amount of alcoholic ingestion sometimes gets rid of stress and stings our life happily. Furthermore, there may be a case where one can hardly deny drinking at a welcome or farewell party. Additionally, it is not always easy to abandon an ordinary habit of drinking even though it has not reached a level of alcohol intoxication yet. Compositions enhancing alcohol metabolism are now under study in order that hangover may be avoided or influences upon the liver may be relieved. For example, γ-aminobutylic acid (GABA) and sesamin extracted from sesame are said to have an effect of enhancing alcohol metabolism. However, it cannot be said that a sufficient effect is obtained from these metabolism enhancing agents.

On the other hand, the inventors have continuously studied effects of theanine which is an amino acid characteristic of green tea. The inventors have found that theanine has various effects of restraining provocation of anxiety, premenstrual syndrome (PMS) and smoking and improving mind concentration and the like. JP-A-H12-143508 and JP-A-H14-97136 disclose some of these found effects. Further, JP-A-H06-40901 discloses an effect of restraining acetaldehyde toxicity.

However, no detailed research has hitherto been made about theanine's enhancement of alcohol metabolism.

SUMMARY OF THE INVENTION

Therefore, an object of the present invention is to provide an alcohol-metabolism enhancing composition containing theanine and ingesta containing the composition.

The inventors made researches on theanine to overcome the aforesaid problem. The inventors found that theanine had an effect of enhancing alcohol metabolism and basically completed the present invention.

The present invention provides an alcohol-metabolism enhancing composition characterized by containing theanine.

The invention further provides ingesta characterized by containing the composition.

The alcohol-metabolism enhancing composition of the invention which will sometimes be referred to as "composition" hereinafter can be used at one time or routinely for the purpose of easing or improving troubles due to ingestion of various types of alcoholic beverages such as hangover or alcoholic hepatic insufficiency. The alcohol-metabolism enhancing effect of the composition is produced as a reduction in the blood alcohol concentration. More specifically, when one ingests the composition of the invention before, during or after ingestion of alcoholic beverage, the blood alcohol concentration based on absorption of alcohol into the body is quickly reduced. Consequently, the troubles due to ingestion of alcoholic beverage can be eased or improved.

The composition of the invention may be contained in ingesta or food or beverage. Accordingly, when the composition is contained in tidbits, meal, drinks or the like, alcohol metabolism can be enhanced easily and reliably.

BRIEF DESCRIPTION OF THE DRAWINGS

Other objects, features and advantages of the present invention will become clear upon reviewing the following description of the embodiments with reference to the accompanying drawings, in which:

FIG. 1 is a graph showing changes in the blood alcohol concentration with time after ingestion of alcohol, wherein symbol "0" designates administration of only ethanol whereas symbol "□" designates combined use of ethanol and theanine;

FIG. 2 is a graph showing changes in liver lipid peroxide with time after ingestion of alcohol;

FIG. 3 is a graph showing changes in liver GSH concentration with time after ingestion of alcohol;

FIG. 4 is a graph showing liver ADH activation (left) and gene CYP2E1 activities (right) after ingestion of alcohol; and

FIG. 5 is a graph showing liver ALDH activity after ingestion of alcohol.

FIG. 6 is a graph showing GTP activity in the blood after ingestion of alcohol for ten days.

FIG. 7 is a graph showing GSH concentrations in the liver after ingestion of alcohol for ten days.

DETAILED DESCRIPTION OF THE INVENTION

Embodiments of the present invention will be described in detail. However, the technical scope of the invention should not be limited by the following description of embodiments but can be practiced in various modified forms. Furthermore, it is noted that the technical scope of the invention should encompass the scope of equivalence.

The anine used in the invention is a glutamic acid derivative contained in tea leaves and a principal component of deliciousness of tea. Theanine is used as a food additive for use as gustatory. Methods of producing theanine used in the invention include a method of extracting theanine from tea leaves, a method of obtaining theanine by organic synthesis reaction (Chem. Pharm. Bull., 19(7) 1301-1307 (1971), a method of obtaining theanine by causing glutaminase to react to a mixture of glutamine and ethylamine (JP-B-H07-55154), a method of culturing cultured cells of tea in culture medium containing ethylamine and enhancing growth of cultured cells while an amount of theanine accumulated in the cells is increased (JP-A-H05-123166), a method of obtaining theanine by substituting an ethylamine derivative such as ethylamine hydrochloride for ethylamine as in JP-B-H07-55154 or JP-A-H05-123166, for example. Theanine may be produced by any one of these methods or another method. Green tea, oolong tea, black tea or the like may be exemplified as tea leaves.

Any one of L-, D- and DL-theanine may be used. L-theanine is preferable since it is particularly recognized as food additives and is economic in use.

A manner, the number of times, a period of administration of the composition of the invention should no be limited. The composition can be administered to a man in a suitable administration manner or preferably by oral administration at once or a plurality of times. Furthermore, when the composition of the invention is ingested at one time or routinely, troubles due to ingestion of various types of alcoholic beverages can be eased or improved. In particular, it is preferable to ingest the composition of the invention before, during or after ingestion of alcoholic beverage.

Theanine used in the invention has a high security. For example, in an acute toxicity test with use of mice, no mice died and abnormality was found in an ordinary state, weight and the like even in the case of oral administration of theanine by 5 g/kg. Furthermore, theanine is known as a principal component of deliciousness of tea and used as a food additive for use as gustatory. An amount of theanine to be added is not limited under the Food Sanitation Law. Moreover, differing from conventional medical substances, theanine has no adverse side effect. Consequently, the composition of the invention can be used as a safe and effective alcohol-metabolism enhancing composition.

As described above, there is no upper limit in an amount of theanine from the standpoint of safety. However, from the standpoint of economy, an amount of theanine to be actually ingested per time ranges from 0.01 mg/kg per weight to 100 mg/kg per weight. An amount of theanine to be ingested preferably ranges from 0.1 mg/kg per weight to 80 mg/kg per weight. An amount of theanine to be ingested more preferably ranges from 1 mg/kg per weight to 50 mg/kg per weight. Theanine used in the invention may be a refined product (containing 95% theanine or more), coarse product (containing 50% to 98% theanine), extract (containing 10% to 50% theanine) or the like.

The composition of the invention may be contained in ingesta or food or beverage. There is no specific limitation to such ingesta. However, for example, the ingesta may include solid food such as dried food containing theanine and liquid food such as .supplement, refreshing drinks, mineral water, favorite beverage and alcoholic drinks.

The solid food may include pastes, soybean-processed food, mousse, jelly, yogurt, frozen dessert, candy, chocolate, chewing gum, cracker, biscuit, cookie, cake and bread.

The liquid food may include tea such as green tea, oolong tea, black tea and herb tea, syrup, concentrated juice, concentrated reduced juice, straight juice, fruit juice, granule-containing fruit juice, fruit juice containing beverage, fruit-vegetable-mixed juice, vegetable juice, carbonated drink, refreshing drink and lactic acid beverage.

Furthermore, it is particularly effective to ingest the composition of the invention before, during or after ingestion of alcoholic beverage (beer, Japanese "sake," wine, distilled rice sprits or Japanese "shochu," whiskey, brandy, etc.). Accordingly, ingesta containing the composition of the invention may preferably include health drink, food (confectionery such as chewing gum and candy, tidbits such as cheese, for example), alcoholic beverage itself (sparkling wine, cocktail, Japanese "chuhai" cocktail, for example). Particularly when the composition of the invention is contained in an alcoholic beverage itself, one ingests the alcohol-metabolism enhancing composition while drinking alcohol. As a result, the invention can provide an alcohol beverage which can prevent hangover.

Furthermore, the composition of the invention can be used with materials such as herbal medicine, herb, amino acid, vitamin, mineral and other materials allowed for use with food. There is no specific limitation to such herbal medicine.

However, for example, the herbal medicine may include Valeriana fauriei, Angelicae radix, Paeoniae radix, Paeonia suffruticosa and ginseng.

There is no specific limitation to the herb. However, for example, the herb may include anise, carrot seed, cloves, coriander, cypress, cinnamon, juniper, ginger, sweet orange, basil, patchouli, bitter orange, fennel, black pepper, bay, peppermint, bergamot, mandarin, myrrh, lemon grass, rosemary, vanilla, hyssop, eucalyptus, lime, lemon, ylangylang, cardamom, clarysage, jasmine, geranium, Bulgarian rose, rose, olibanum, matricaria, sandalwood, verbena, petit grain, vetivera zizanoides, marjoram, Melissa officinelis and rosewood. Peppermint is more preferable. These herbs may be extract, essential oil, herb tea or the like as its form.

There is no specific limitation to the amino acid. However, for example, the amino acid may include glutamine, glutamine acid, inosinic acid, alanine, arginine, asparaginic acid, threonine, serine, taurine, thiotaurine and hypotaurine.

There is no specific limitation to the vitamin. However, for example, the vitamin may include vitamin A, vitamin B1, vitamin B2, vitamin B6, vitamin B12, vitamin C, vitamin D, vitamin E, vitamin K, folic acid, niacin, lipoic acid, pantothenic acid, biotin and ubiquinone. Vitamin B1, B6 and B12 are more preferable. Furthermore, the vitamins include the derivatives thereof.

There is no specific limitation to the mineral. However, for example, the mineral may include calcium, iron, magnesium, copper, zinc, selenium and potassium.

Furthermore, the other material allowed to be contained in food may include aloe, royal jelly, placenta, propolis, isoflavone, soy isoflavone, egg yolk lecithin, lecithin, chondroithin, cacao mass, collagen, vinegar, chlorella, spirulina, ginkgo leaf, green tea, hardy rubber tree, oolong tea, mulberry leaf, Rubus suavissimus, Lagerstroemia speciosa, unsaturated fatty acid, saccharide such as sugar alcohol and oligosaccharide, fungi such as bifidus bacillus, mushrooms such as agaricus, agaricus blazei Murrill, blacket fungus of the genus Fores, Grifola frondose, fruit such as blueberry, prune, grape, olive and plum, molokheiya such as peanut, almonde, sesame and pepper, vegetables such as green pepper, cayenne pepper, welsh onion, pumpkin, gourd, carrot, burdock, molokheiya, garlic, beefsteak plant, Japanese horseradish, tomato, scallion, leaf vegetables, sweet potato and beans, seaweeds such as "wakame" seaweed, fish and shellfish, meat of beast, birds and whales and grains. Furthermore, usable are extracts, dried products, coarse product, refined product, processed product and distilled product.

The invention can further provide an alcohol metabolism enhancing medical supplies containing theanine as an effective component. The medical supplies may include an internal medicine, injection medicine, pasting, suppository and inhalation medicine. However, there is no limitation to them. The internal medicine may include conventionally used tablet, capsulation, powder, granule and drink insecticide. The injection medicine may include intramuscular injection, intracutaneous injection, hypodermic injection and intravenous injection. The pasting medicine may include a mixture comprising a known carrier conventionally used for suppository and effective component of the invention and sheet to which the mixture is applied. The suppository medicine may include a mixture of the composition of the invention and conventionally used glycerogelatin, sodium stearate or propylene glycol monostearate. The inhalation medicine may include one having such a formulation as to be absorbed through nare or buccal cavity into the body with moisture or air in a conventional inhalation manner, for example.

The composition of the invention may be used with green tea extract. The green tea extract may include 0.001% to 90% catechin (A) or more preferably, 0.01% to 85% catechin (A) or further more preferably 0.1% to 80% catechin (A). The catechin (A) contained in green tea is a general term for non-epicatechin (B) such as catechin, gallocatechin, catechin gallate and garocatechin gallate and epicatechin (C) such as epicatechin, epigallocatechin and epigallocatechin gallate (A=B+C). Furthermore, a weight ratio (B/C) of non-epicatechin (B) to epicatechin (C) ranges from 0.25 to 9.0 or more preferably, from 0.43 to 9.0 or further more preferably from 0.43 to 5.67. An amount of green tea extract per ingestion ranges from 0.0005 mg/weight by kg to 10000 mg/weight by kg or more preferably, from 0.01 mg/weight by kg to 1600 mg/weight by kg or further preferably, from 1 mg/weight by kg to 100 mg/weight by kg. Furthermore, when theanine (A) and green tea extract (D) are used together, a weight ratio (D/A) of them ranges from 0.05 to 100 or more preferably, from 0.1 to 20 or further more preferably, from 0.1 to 2.

A method of manufacturing a composition of the invention is not limited only if the method includes a step of blending theanine, for example. For example, the method may include an ordinary method of manufactuting food or medicines such as one of blending theanine, obtaining a mixed solution by dissolving theanine in a solvent, freezing and drying the mixed solution and spray drying the mixed solution.

The product of the invention may be, in a form, a solution, suspended substance, powder or solid but should not be limited to them. The food may include condiment, soup, coffee, cocoa, dairy or milk product, sparkling wine, cocktail, Japanese "chuhai" cocktail. The medicines may include tablet, capsulation and injection medicine comprising any known carrier suitably selected according to the usage, formula and the like, the composition of the invention and other amalgamations.

Embodiments

The invention will be described in more detail by way of embodiment. However, the scope of the invention should not be limited to the embodiments. In the following description, symbol mg/kg designates an amount of substance ingested by mg per weight.

Example 1: Manufacturing theanine by an enzyme method

0.3 M glutamine and 1.5 M methylamine hydrochloride were reacted in the presence of 0.3 U glutaminase (commercially available) at 30°C for 22 hours in a buffer solution of 0.05 M boric acid (pH 11), whereby 225 nm theanine was obtained. Reaction liquid was applied to Dowex $50 \times 8$ columnar chromatography and Dowex $1 \times 2$ columnar chromatography (both made by Muromachi Chemical Co., Ltd.) thereby to be processed by ethanol, whereby an object substance is isolated from the reaction liquid. As a result, 8.5 g theanine was obtained.

The isolated substance was applied to an amino acid analyzer (made by Hitachi Co.) and paper chromatography. Since the isolated substance behaved in the same way as a standard substance, it was recognized as L-theanine. When the isolated substance was processed by hydrolysis using hydrochloric acid or glutaminase, glutamine acid and ethylamine were produced in a ratio of 1:1. Thus, since the isolated substance was hydrolyzed by glutaminase, it was shown that ethylamine was γ-ethylamine of glutamine acid. Furthermore, it was confirmed on the basis of glutamate dehydrogenase that glutamine acid produced by hydrolysis was L-glutamine acid.

Example 2: Extraction of theanine from tea leaves

10 kg tea leaf (Camellia sinensis) was extracted using heated water. Extract was concentrated and divided to be isolated to a catechin layer and a water layer. A solvent of the water layer is left in vacuum, so that the obtained extract was passed through a cation exchange resin (type NTR 729 HF made by Nitto Denko Corporation). Resin was cleaned by water and thereafter, washed by aqueous ammonia to be left in vacuum. After water had been added to the extract, it was spray dried such that 125 g of 20% theanine was obtained.

Example 3: Extraction of theanine from tea leaves

10 kg tea leaf (Camellia sinensis) was extracted using heated water and thereafter, the obtained extract was passed through a cation exchange resin (type HCR W-2 made by Muromachi Chemical Industry Co., Ltd.) so as to be eluted by 1N NaOH. Eruted fraction was passed through activated charcoal (Taiko activated charcoal SG made by Futamura Chemical Industry Co., Ltd. The fraction eruted by 15% ethanol was concentrated using an RO film (type NTR 729 HF made by Nitto Denko Corporation). The concentrated eruted fraction was refined by columnar chromatography and then re-crystallized such that 24.8 g theanine was obtained.

Theanine (product name: Suntheanine made by Taiyo Kagaku Co., Ltd.) was used in experiments and manufacture of composition.

Embodiment 1: Improvement in activity of alcohol-metabolism enzyme

Experimental method:

Ethanol was ingested to $CDF_1$ male mice 3.0 g/weight by kg by oral administration. Theanine was ingested to the abdominal cavity of each mouse once before ingestion of ethanol. Concentration of ethanol in the blood and concentration of lipid peroxide in the liver were measured. GSH concentration was also measured. Furthermore, alcohol dehydrogenase, aldehyde dehydrogenase and enzyme activity of cytochrome P2E1 were also measured.

Measuring methods:

The concentration of ethanol in the blood was measured by the following ADH method. 0.1 mL of blood was added to 0.8 mL of 0.33 N perchloric acid. After execution of a vortex process, a centrifugal process was carried out under the condition of 1200 g for 5 minutes. 0.1 mL of supernatant was mixed with 0.1 mL of buffer liquid containing 4.8 mL of sodium phosphate /semicarbazide buffer liquid (pH8.7) and 0.48 mM of NAD. 0.02 mL of ADH (≥32IU/mL) was further added to the mixture and incubated at 37°C for 25 minutes. Thereafter, absorbance was measured at 340 nm. An unknown sample concentration was calculated by the comparison of ethanol concentration with known standard sample data.

The lipid peroxide concentration in the liver was measured in the following method (TBA fluorometry). 0.1 mL of liver sample (2% homogenate in physiological salt solution) was added with 0.5 mL of 3% SDS, 1.5 mL of 2.0 M acetic acid buffer liquid (pH3. 6), 1.5 mL of 0.8% thiobarbituric acid and 0.4 mL of purified water so that a total amount was 4.0 mL. In a standard sample, 20 µL of 50 µmol/L malondialdehyde (MDA) was added instead of the above liver sample and 0.48 purified water was added. The solution was heat-treated in boiling water for 75 minutes and thereafter, cooled for 5 minutes. 1.0 mL of 0.2 N chloride and 5.0 mL of n-butanol were mixed to the cooled substance and thereafter, shaken for 30 seconds. 1200 g of substance was centrifugally processed for 15 minutes. Supernatant was sampled and fluorometry (excitation wavelength: 515 nm; and fluorescent wavelength: 553 nm) was carried out. The unknown sample was calculated using calibration curve.

The GSH concentration in the liver was measured by the following method (HiSSIN-Hilf). 5% homogenate of the liver or heart was prepared in a 0.1 M sodium phosphate-0. 005 M EDTA buffer liquid (pH 8.0) using a potter type Teflon homogenizer while being iced. 0.25 mL of 25% metaphosphoric acid was added to 0.75 mL of homogenate liquid and mixed well. 10000 g of substance was centrifugally processed for 30 minutes. Thereafter, supernatant was sampled and diluted by 50 times with 0.1 M sodium phosphate-0.005 M EDTA buffer liquid (pH 8.0). To 0.20 mL of the diluted liquid was added 3.6 mL of 0.1 M sodium phosphate-0.005 M EDTA buffer liquid (pH 8. 0) and 0.20 mL of 0.1% o-phthalaldehyde (OPT)-methanol liquid. The mixture was left at a room temperature for 5 minutes and thereafter, the fluorometry (excitation wavelength: 350 nm; and fluorescent wavelength: 420 nm) was carried out. The unknown sample was calculated using calibration curve.

The alcohol dehydrogenase was measured by the following method (a method improved by Haseba et al.). 10% homogenate liquid of the liver was prepared in a 0. 5 M tris-hydrochloric acid buffer liquid (pH 8.5) while being iced. Thereafter, 105000 g of the homogenate liquid was centrifugally isolated at 4°C for 20 minutes. Supernatant was also centrifugally isolated at 4°C for one hour such that resultant supernatant was sampled for measurement. 0.1 mL of sample was put in a cell in which temperature was maintained at 37°C and diluted with 2.0 M glycine -sodium hydroxide buffer liquid (pH 10.7). Thereafter, 0.1 mL of 39 mM NAD was added to the diluted sample, which was previously incubated at 37°C for one minute. 0.1 mL of 2% ethanol serving as a substrate was added. The sample was further incubated at 37°C for three minutes. In this while, absorbance was measured at 340 nm. Enzyme activity was obtained from the difference of absorbance in compliance with the following equation:

```
ADH activity [µ mol/min·mg protein

= 0.40 × ΔA/min × 1/0.1 × 1/protein (mg/ml)

ΔA Sample/min - ΔA Blank/min = ΔA/min                           (1)
```

The aldehyde dehydrogenase converts aldehyde to carboxylic acid such as acetic acid. Enzyme activity was measured by the following method (method by Manthey et al).

5% homogenate liquid of the liver was prepared in a 0.1 M phosphoric acid buffer liquid (pH 7.5) while the liver was being iced. Thereafter, 15000 g of the homogenate liquid was centrifugally isolated at 4°C for 30 minutes. Supernatant was sampled for measurement. To 0.2 mL of sample was added 1.7 mL of cocktail (a mixture of 64 mM of sodium pyrophosphate, 0.08 mL of 100 mM NAD, 0.02 mL of 10 mM pyrazole, 0.2 mL of 10 mM EDTA and 0.4 mL of distilled water). The cocktail was previously incubated at 37°C for three minutes. 0.1 mL of 80 mM acetaldehyde was added to the cocktail, which was further incubated at 37°C. Absorbance was measured at 340 nm. Enzyme activity was obtained from the difference of absorbance in compliance with the following equation:

```
ALDH activity [µ mol/min·mg protein

= 1000 × 6220 × ΔA/min × 2.0/0.2 × 1/protein (mg/ml)

ΔA Sample/min - ΔA Blank/min = ΔA/min                           (2)
```

Chemical solution metabolism enzyme activity of cytochrome P2E1 (CYP2E1) was measured by an HPLC method in which the substrate and diluted microsome liquid were reacted and produced metabolite was measured using a fluorescence detector. Regarding a liver sample, 20% homogenate liquid was prepared in a 0.25 M sucrose and 50 mM tris hydrochloric acid buffer liquid (pH 7.4) using a potter type Teflon homogenizer while being iced. Thereafter, 10000 g of the homogenate liquid was centrifugally isolated at 4°C for 30 minutes. Supernatant was fractionated as cytosol fraction and used for the GST measurement. 1.0 mL of 50 mM tris hydrochloric acid buffer liquid (pH 7.4) was added to microsome fraction after the centrifugal isolation and suspended again to be used for measurement of enzyme activity. For activity measurement, triple-volume 50 mM tris hydrochloric acid buffer liquid (pH 7.4) was added and diluted quadruply. Furthermore, the concentration of protein in the microsome fraction was obtained so that activity per amount of protein was measured apart from enzyme activity measurement.

Enzyme activity was measured in the following method. With 20 $\mu$L microsome fraction were mixed 380 $\mu$L of substrate solution (a mixture of 375 $\mu$L of 67 mM potassium phosphate buffer liquid (pH6.8), 2.5 $\mu$L of 4 mM para-nitrophenol-methanol solution and 2.5 $\mu$L refined water) and 50 $\mu$L refined water. The mixture was previously incubated at 25°C for five minutes and was thereafter mixed with 50 $\mu$L NADPH-magnesium chloride solution (only magnesium chloride solution as a blank). The mixture was further incubated at 37°C for 10 minutes. After completion of reaction, 25 $\mu$L trifluoroacetic acid was further mixed with the mixture and was shaken and thereafter, incubated at 0°C for 15 minutes so that 12000 g of mixture was centrifugally isolated for 15 minutes, whereby supernatant was analyzed by HPLC. In the HPLC analysis, para-nitrocatechol produced by enzyme reaction was measured. Regarding the HPLC conditions, Capcell Pack UG80 (5 $\mu$m, 250$\times$4.6 mm, made by Shiseido) was used as a column and trifluoroacetic acid-acetonitrile-water (0.1:25:74.9, v/v) was used as a moving phase at flow velocity of 0.8 mL/min. The temperature of the column was set at 26°C. ECD (+700 mV vs silver/silver chloride)

Experimental results

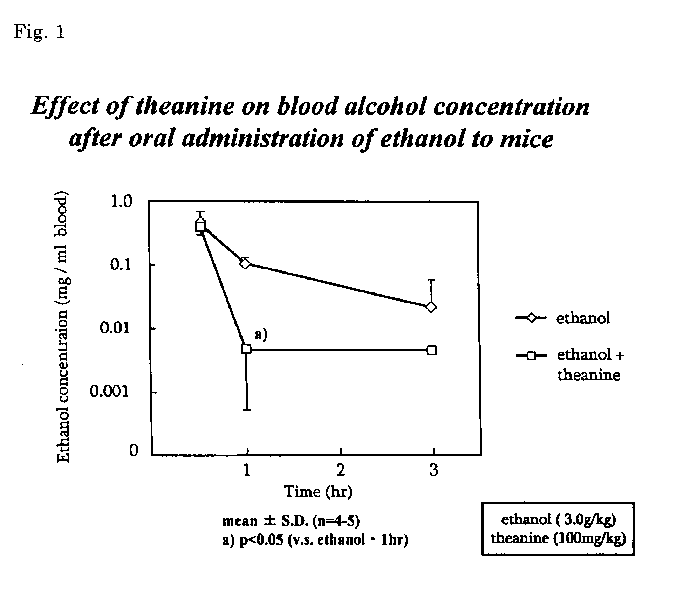

TABLE 1 and FIG. 1 show changes in alcohol concentration in the blood after ingestion of alcohol to the mice.

TABLE 1

| Ethanol concentration in the blood (mg/ml) | | | | | |
|---|---|---|---|---|---|
| Ingested medicine | | Time (h) | | | |
| | | 0 | 0.5 | 1 | 3 |
| Ethanol | Mean | 0 | 0.498 | 0.108 | 0.022 |
| | S. D. | 0 | 0.290 | 0.013 | 0.006 |
| Ethanol + theanine | Mean | 0 | 0.498 | 0.004 | 0.004 |
| | S. D. | 0 | 0.290 | 0.002 | 0.000 |

When theanine (100 mg/kg) was used 30 minutes after ingestion of ethanol (3.0 g/kg), the ethanol concentration in the blood was significantly reduced (p<0.05)after one hour as compared with the case where only ethanol (3.0 g/kg) was ingested.

Furthermore, $AUC_{0-3hr}$ was reduced by 76% by the joint use of theanine as compared with the case where only ethanol was ingested. Consequently, it was found that disappearance of ethanol from the body was enhanced by the joint use of ethanol and theanine.

TABLE 2

| Lipid peroxide concentration (mg/ml) | | | | | | | |
|---|---|---|---|---|---|---|---|
| Ingested medicine | | Time (h) | | | | | |
| | | 0 | 0.5 | 1 | 2 | 3 | 5 |
| Ethanol | Mean | 0.617 | 0.573 | 0.565 | 0.580 | 1.036 | 0.568 |
| | S. D. | 0.072 | 0.016 | 0.121 | 0.093 | 0.157 | 0.132 |
| | (%) | 100 | 93 | 92 | 94 | 168 | 92 |
| Ethanol+ theanine | Mean | 0.617 | 0.573 | 0.476 | 0.504 | 0.449 | 0.599 |
| | S. D. | 0.072 | 0.016 | 0.046 | 0.049 | 0.061 | 0.089 |
| | (%) | 100 | 93 | 77 | 82 | 73 | 97 |

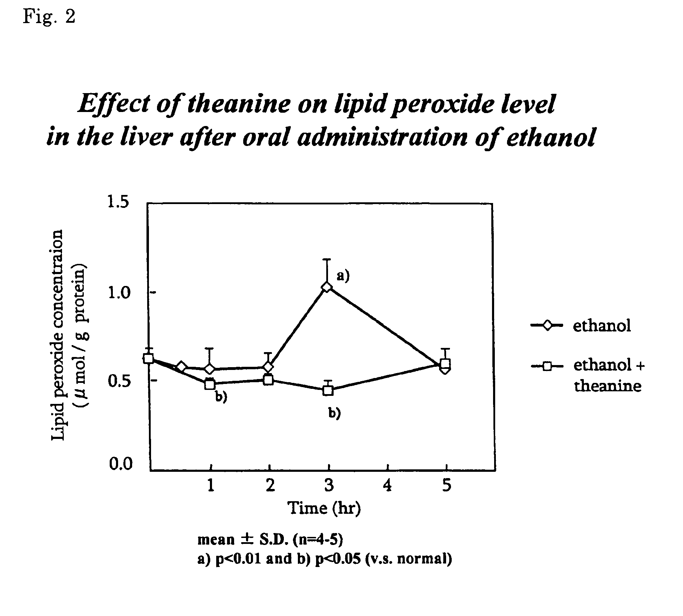

TABLE 2 and FIG. 2 show changes in lipid peroxide which is increased in hepatopathy due to ethanol.

When only ethanol was ingested, lipid peroxide was increased to 1.036 (in the unit of $\mu$ mol/g protein). This value corresponds to 168% of the value in the normal state (p<0.005). On the other hand, when ethanol and theanine were jointly used, the lipid peroxide concentration was significantly reduced after one and three hours as compared with the normal state (p<0.005). Consequently, it was found that theanine restrains a temporary increase in lipid peroxide concentration due to ingestion of ethanol, maintaining the normal level.

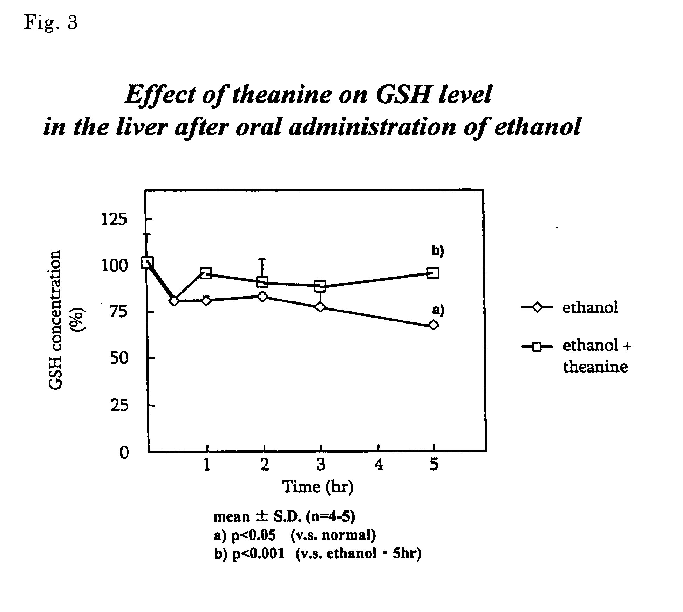

TABLE 3 and FIG. 3 show changes in glutathione concentration after ingestion of ethanol.

TABLE 3

| Ingested medicine | | GSH concentration (%) | | | | | |
|---|---|---|---|---|---|---|---|
| | | Time (h) | | | | | |
| | | 0 | 0.5 | 1 | 2 | 3 | 5 |
| Ethanol | Mean | 100.00 | 79.84 | 78.83 | 81.57 | 75.96 | 65.50 |
| | S. D. | 16.42 | 0.98 | 3.64 | 3.65 | 9.49 | 1.89 |
| Ethanol+ Theanine | Mean | 100.00 | 79.84 | 94.73 | 89.82 | 87.07 | 93.93 |
| | S. D. | 16.42 | 0.98 | 0.11 | 12.52 | 0.01 | 0.82 |

When only ethanol was ingested, glutathione concentration was gradually reduced and was significantly reduced to 65.5% 5 hours after ingestion as compared with the normal state ($p < 0.05$). On the other hand, when ethanol and theanine were jointly used, the glutathione concentration was temporarily reduced 30 minutes after ingestion (79.84% of that in the normal state). Thereafter, the glutathione concentration rapidly returned nearly to the normal value. Furthermore, the glutathione concentration was significantly increased 5 hours after ingestion as compared with the case where only the ethanol was ingested ($p < 0.001$). Consequently, it was found that ingestion of theanine prevented hyperoxidation due to ethanol metabolism, thereby protecting the liver.

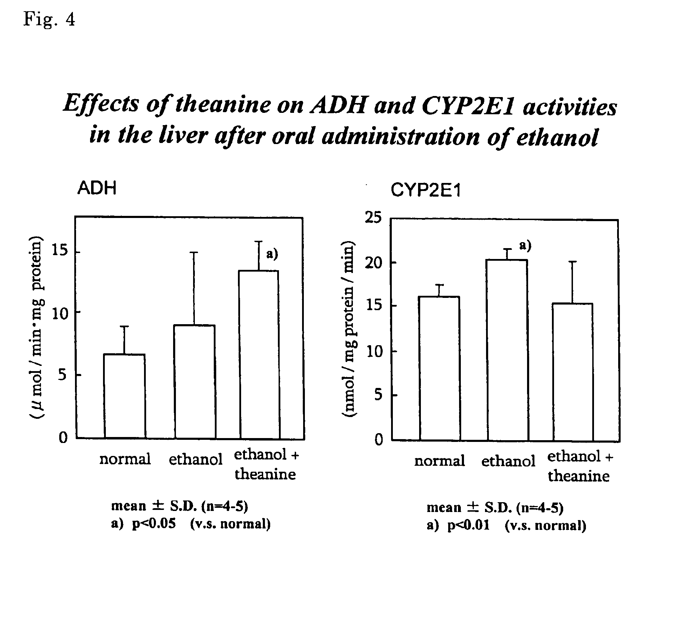

TABLES 4 and 5 and FIG. 4 show changes in alcohol dehydrogenase activity (ADH) CYP2E1 in the liver 3 hours after ingestion of ethanol.

TABLE 4

| ADH activity ($\mu$mol/min·mg protein)(3 hours) | | |
|---|---|---|
| Ingested medicine | Mean | S.D. |
| Control | 7.39 | 2,76 |
| Ethanol | 9.13 | 5.94 |
| Ethanol+Theanine | 13.56 | 2.35 |

TABLE 5

| CYP2E1 (nmol/mg protein/min)(3 hours) | | |
|---|---|---|
| Ingested medicine | Mean | S.D. |
| Control | 16.1 | 1.4 |
| Ethanol | 20.4 | 1.3 |
| Ethanol+Theanine | 15.4 | 4.9 |

ADH activity was increased to about 123% by ingestion of only ethanol as compared with the case of control (normal state), whereupon enzyme was induced such that activity was increased. On the other hand, when ethanol and theanine were jointly used, the ADH activity was further increased (about 183% of that of control), whereupon enzyme activity was increased to a large degree.

Furthermore, CYP2E1 performing part of ethanol metabolism was also measured. This protein acts when excessive ethanol is present. Thus, the protein is considered to be induced by chronic ingestion of ethanol. Since a large amount of active oxygen is produced in the alcohol metabolism by CYP2E1, it is obvious that cytotoxicity appears. Tables and figures show that CYP2E1 activity was significantly increased by the ingestion of only ethanol as compared with control ($p < 0.01$). On the other hand, when ethanol and theanine were jointly used CYP2E1 activity was slightly reduced (about 95.7% of that of control). Consequently, it was found that there was a possibility of avoiding hepatopathy via CYP2E1 activity.

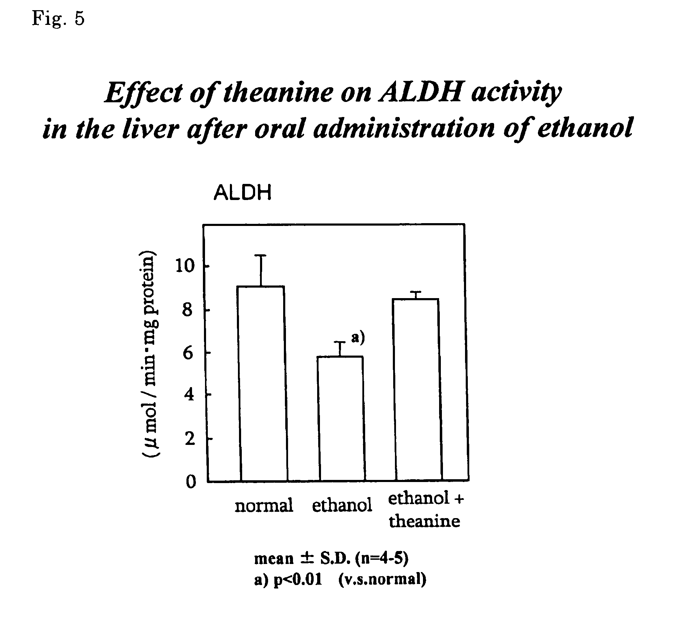

TABLE 6 and FIG. 5 show changes in aldehyde metabolism enzyme activity (ALDH) in the liver 3 hours after ingestion of ethanol.

TABLE 6

| ALDH activity (μmol/min·mg protein) (3 hours) | | |
|---|---|---|
| Ingested medicine | Mean | S.D. |
| Control | 9.13 | 1.50 |
| Ethanol | 5.80 | 0.82 |
| Ethanol+Theanine | 8.47 | 0.51 |

ALDH was reduced to about 63.5% as compared with control when only ethanol was ingested (p<0.01). On the other hand, when ethanol and theanine were jointly used, ALDH presented substantially the same activity as control (about 92.8%). In the ethanol metabolism in the body, aldehyde oxidation process was rate-limiting. From the above results, it was found that theanine increased ALDH activity. Accordingly, aldehyde metabolism was also enhanced such that alcohol metabolism was enhanced by theanine as well as by ADH activity.

Embodiment 2: The effects of chronic administration of theanine on the alcoholic liver injury

Experimental method:

Ethanol was ingested to $CDF_1$ male mice (5 weeks old) 1.0 g/kg-weight or 2.0 g/kg-weight by oral administration twice in a day. Theanine was ingested to the abdominal cavity of each mouse 100 mg/kg-weight. After ingestion of ethanol and theanine for ten days, concentrations of GOT, GPT, and γ-GTP in the blood were measured. Concentrations of lipid peroxide and GSH in the liver were measured.

Measuring methods:

The concentrations of GOT and GTP in the blood were measured by using the TRANS AMYLASE C-test WAKO (WAKO Pure Chemical Industries, Ltd). The concentration of γ-GTP was measured by using the γ-GTP C-test WAKO (WAKO Pure Chemical Industries, Ltd). Concentrations of lipid peroxide and GSH in the liver were measured by the method written in Embodiment 1.
The concentration of GOT was measured by the following method. 0.02 mL of serum was added to 0.5 mL of GOT-measuring-substrate-buffer and incubated at 37°C for 5 minutes. Thereafter, 0.5 mL of coloring reagent was added, incubated at 37°C for 20 minutes. 2.0 mL of stop solution was added to the mixture. Thereafter absorbance was measured at 555 nm. GOT activity (Karmen unit) in the blood was calculated by the comparison of GOT activity with known standard sample data plot.
The concentration of GPT was measured by the following method. 0.02 mL of serum was added to 0.5 mL of GPT-measuring-substrate-buffer and incubated at 37°C for 5 minutes. Thereafter, 0.5 mL of coloring reagent was added, incubated at 37°C for 20 minutes. 2.0 mL of stop solution was added to the mixture. Thereafter absorbance was measured at 555 nm. GPT activity (Karmen unit) in the blood was calculated by the comparison of GPT activity with known standard sample data plot.
The concentration of γ-GTP was measured by the following method. After substrate buffer was incubated at 37°C for 3 minutes, 0.02 mL of serum was added and incubated at 37°C for 15 minutes. Thereafter, 2.0 mL of coloring reagent was added. Absorbance was measured at 660 nm. γ-GTP activity (IU/L, 37°C) in the blood was calculated by the comparison of γ-GTP activity with known standard sample data plot.

Experimental results

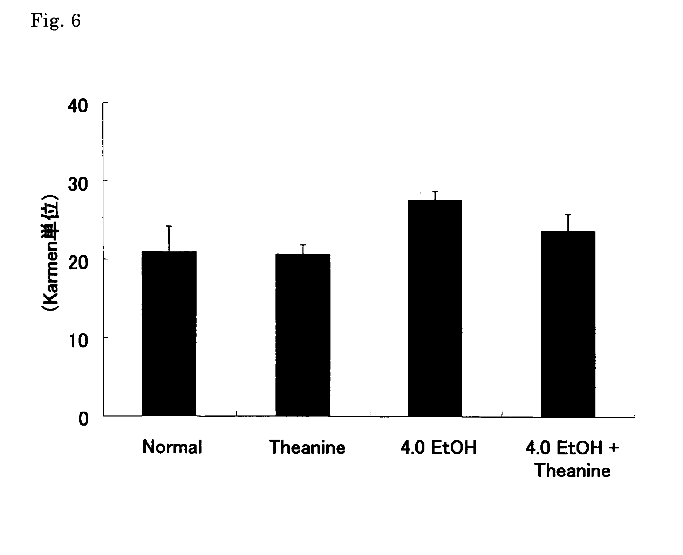

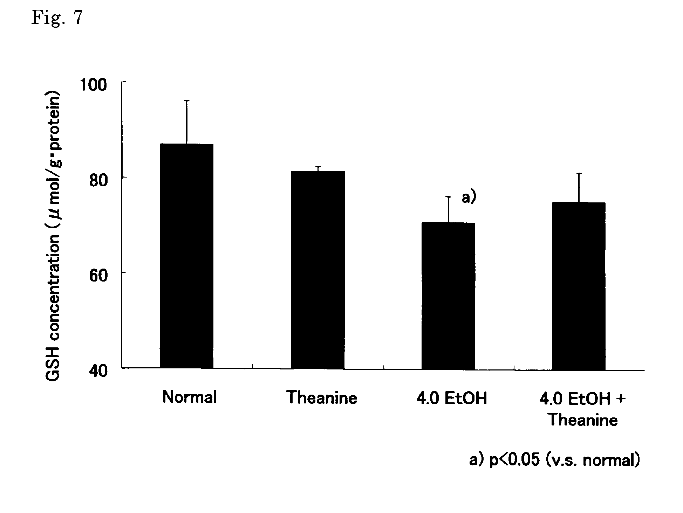

FIG. 6 and FIG.7 show the results of theanine effects on the alcoholic liver injury. FIG. 6 showed that the GPT concentrations in the blood were increased by alcohol administration, and the increase of the GPT concentrations could be reduced by theanine. The same tendency was shown with the GOT concentrations in the blood. The increase of lipid peroxide in the liver would be reduced by theanine. Though the GSH concentrations in the liver was decreased by alcohol, the decrease of the GSH could be reduced by theanine (FIG.7).These results show that theanine reduces the alcohol liver injury.
According to the foregoing embodiment, theanine increases both ADH activity and ALDH activity, enhancing alcohol metabolism such that alcohol concentration in the blood can quickly be reduced. Furthermore, increase in CYP2E1 is suppressed such that hepatopathy due to active oxygen can be prevented.
Theanine reduces the alcohol liver injury.
Consequently, the composition containing theanine can ease or improve troubles (overhang, hepatopathy due to alcohol, for example) caused by ingestion of various types of alcohol beverages.

Based on the above-described findings, the composition containing theanine or ingestion containing the composition can be provided as follows.

Embodiment 3

Materials as shown in TABLE 7 were mixed together and made into tablets, whereby tablets containing theanine were manufactured (1000 mg per tablet).

TABLE 7

| Material | Weight % | Weight |
|---|---|---|
| Theanine | 5.0 | 50.00 |
| Chamomile | 50 | 500 |
| Enzyme catalized guar gum | 1.7 | 17.00 |
| Crystalline cellulose | 0.75 | 7.50 |
| Reduced maltose starch syrup powder | 5.0 | 50.0 |
| Lactose | 36.675 | 366.75 |
| Silicon dioxide | 0.125 | 1.25 |
| Sucrose fatty ester | 0.75 | 7.50 |
| Total | 100 | 1000 |

Embodiment 4

Candy containing theanine was made from materials as shown in TABLE 8.

TABLE 8

| Material | Weight |
|---|---|
| Granulated sugar | 41 kg |
| Starch syrup | 23 kg |
| Theanine | 10 kg |
| Chamomile | 60 kg |
| Perfume (lemon flavor) | 0.05 kg |
| 50% tartaric acid | 1 kg |
| Water | 30 kg |

Granulated sugar was heated to 110°C while dissolving in 20 kg water. The remaining 10 kg water in which theanine is dissolved, chamomile, starch syrup were added to the granulated sugar and the temperature was increased to 110°C. Heating was interrupted and 50% tartaric acid was further added to the mixture. The mixture was cooled to a range from 75°C to 80°C and then shaped by a shaping roller, whereby candy containing theanine was prepared. The theanine contained in the candy was 89.6 mg/g where one candy weighs 1.2 g.

Embodiment 5

A beverage containing theanine was made using materials as shown in TABLE 9.

TABLE 9

| Material | Weight |
|---|---|
| Fructose and dextrose | 12 kg |
| Blue-berry syrup | 1 kg |
| 1/5 transparent lemon juice | 0.4 kg |
| Sodium acid citrate | 0.05 kg |
| 50% sodium acid citrate (crystal) | for pH adjustment |

(continued)

| Material | Weight |
|---|---|
| Theanine | 0.1 kg |
| Chamomile | 0.6 kg |
| Perfume (blue berry flavor) | 0.05 kg |
| Water | a proper quantity |
| Total | 100 kg |

To water were added fructose and dextrose, blue-berry syrup, 1/5 transparent lemon juice, sodium acid citrate, chamomile and theanine. The materials were agitated and dissolved. 50% sodium acid citrate (crystal) was used so that the materials were prepared so as to have pH 3.1. The temperature was increased to 95°C and thereafter, perfume was added. 100 mL of the mixture was cooled so that a blue berry beverage containing theanine was made. Theanine contained in the blue berry juice was 98.3 mg/100 mL.

Embodiment 6

A beverage containing theanine was made using materials as shown in TABLE 10.

TABLE 10

| Material | Weight |
|---|---|
| Fructose and dextrose | 6 kg |
| Theanine | 0.1 kg |
| Chamomile | 0.6 kg |
| Pyrophosphoric acid ferric iron | 0.06 kg |
| Placenta extract | 0.01 kg |
| 100% grape fruit syrup | 30 kg |
| Sodium acid citrate | for pH adjustment |
| Perfume (grape fruit flavor) | 0.05 kg |
| Water | a proper quantity |
| Total | 100 kg |

To water were added sodium acid citrate, theanine, chamomile, pyrophosphoric acid ferric iron, placenta extract and 100% grape fruit syrup. The materials were agitated and dissolved. Sodium acid citrate was used so that the materials were prepared so as to have pH 3.1. The temperature was increased to 95°C and thereafter, perfume was added. 100 mL of the mixture was cooled so that a grape fruit beverage containing theanine was made. Theanine contained in the grape fruit juice was 96.4 mg/100 mL.

Embodiment 7

An alcohol beverage ("chuhai" containing 7% alcohol) containing theanine was made using materials as shown in TABLE 11.

TABLE 11

| Material | Blended amount by g |
|---|---|
| Fructose and dextrose | 5.00 |
| 1/5 lemon syrup | 1.00 |
| Shochu (containing 25% alcohol) | 28.00 |
| Sodium acid citrate (crystal) | 0.20 |
| Sodium acid citrate | 0.05 |
| Lemon essence | 0.10 |
| Water | Remainder |

(continued)

| Material | Blended amount by g |
|---|---|
| Theanine (Suntheanine, made by Taiyo Kagaku Co., Ltd.) | 0.2 |
| Carbonated water | 50.00 |
| Total | 100.00 |

When "chuhai" is made from the above materials, 8 materials (fructose and dextrose, 1/5 lemon syrup, shochu (containing 25% alcohol), sodium acid citrate (crystal), sodium acid citrate, lemon essence, water and theanine) except carbonated water are blended and dissolved and contained in cans or the like after cooling. Sufficiently cooled carbonated water is mixed and sealed.

Embodiment 8

20 g whiskey, 5 g rime juice, 2 g syrup, 1 g sugar, 0.2 g theanine (Suntheanine, made by Taiyo Kagaku Co., Ltd.), 72 g mineral water are well blended and sealed, whereby cocktail is obtained.

Embodiment 9

20 mL Vodka, 40 mL grape fruit juice, 0.1 g salt, theanine (Suntheanine, made by Taiyo Kagaku Co., Ltd.) and 5 g Fructose and dextrose are well blended and sealed, whereby cocktail is obtained.

Embodiment 10

Shochu containing 35% alcohol (White liquor), plume and sugar are soaked in a ratio of 3: 3: 1. The plume is removed after three months. Theanine (Suntheanine, made by Taiyo Kagaku Co., Ltd.) is dissolved into clear liquid so that 0.1% is obtained and sealed, whereby plume liquor is obtained.

Each of the embodiments 3 to 10 can achieve the same effect as embodiment 1 and embodiment 2.

The foregoing description and drawings are merely illustrative of the principles of the present invention and are not to be construed in a limiting sense. Various changes and modifications will become apparent to those of ordinary skill in the art. All such changes and modifications are seen to fall within the scope of the invention as defined by the appended claims.

**Claims**

1. An alcohol-metabolism enhancing composition containing theanine.

2. An ingesta containing the composition as claimed in claim 1.

Fig. 1

## *Effect of theanine on blood alcohol concentration after oral administration of ethanol to mice*

mean ± S.D. (n=4-5)
a) p<0.05 (v.s. ethanol · 1hr)

ethanol ( 3.0g/kg)
theanine (100mg/kg)

Fig. 2

## *Effect of theanine on lipid peroxide level in the liver after oral administration of ethanol*

mean ± S.D. (n=4-5)
a) $p < 0.01$ and b) $p < 0.05$ (v.s. normal)

Fig. 3

# *Effect of theanine on GSH level in the liver after oral administration of ethanol*

mean ± S.D. (n=4-5)
a) p<0.05   (v.s. normal)
b) p<0.001   (v.s. ethanol · 5hr)

Fig. 4

## Effects of theanine on ADH and CYP2E1 activities in the liver after oral administration of ethanol

ADH

mean ± S.D. (n=4-5)
a) p<0.05 (v.s. normal)

CYP2E1

mean ± S.D. (n=4-5)
a) p<0.01 (v.s. normal)

Fig. 5

## *Effect of theanine on ALDH activity in the liver after oral administration of ethanol*

ALDH

mean ± S.D. (n=4-5)
a) p<0.01   (v.s.normal)

Fig. 6

Fig. 7

a) p<0.05 (v.s. normal)

## INTERNATIONAL SEARCH REPORT

| | International application No. |
|---|---|
| | PCT/JP2005/008283 |

**A.    CLASSIFICATION OF SUBJECT MATTER**
Int.Cl⁷  A61K31/198, A23L1/305, 2/02, A61P3/00

According to International Patent Classification (IPC) or to both national classification and IPC

**B.    FIELDS SEARCHED**

Minimum documentation searched (classification system followed by classification symbols)
Int.Cl⁷  A61K31/198, A23L1/305, 2/02, A61P3/00

Documentation searched other than minimum documentation to the extent that such documents are included in the fields searched
Jitsuyo Shinan Koho         1922-1996    Jitsuyo Shinan Toroku Koho    1996-2005
Kokai Jitsuyo Shinan Koho   1971-2005    Toroku Jitsuyo Shinan Koho    1994-2005

Electronic data base consulted during the international search (name of data base and, where practicable, search terms used)
BIOSIS(STN), CAOLD(STN), CAplus(STN), EMBASE(STN), MEDLINE(STN),
JSTPlus(JOIS)

**C.    DOCUMENTS CONSIDERED TO BE RELEVANT**

| Category* | Citation of document, with indication, where appropriate, of the relevant passages | Relevant to claim No. |
|---|---|---|
| X | Yasuyuki SATSUKA et al., 'Ryokucha Amino acid Theanine ni yoru Ethanol Taisha Sokushin Sayo', Dai 58 Kai The Japanese Society of Nutrition and Food science Taikai Koen Yoshishu, Heisei 16 Nen 4 Gatsu 1 nichi, pages 251 | 1-2 |
| X | JP 6-40901 A  (Suntory Ltd.), 15 February, 1994 (15.02.94), (Family: none) | 1-2 |

☐  Further documents are listed in the continuation of Box C.        ☐  See patent family annex.

| | |
|---|---|
| *     Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 27 May, 2005 (27.05.05) | 14 June, 2005 (14.06.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H12143508 A
- JP H1497136 A
- JP H0640901 A
- JP H0755154 B
- JP H05123166 A

**Non-patent literature cited in the description**

- *Chem. Pharm. Bull.,* 1971, vol. 19 (7), 1301-1307